# EUROPEAN PATENT APPLICATION

(11) **EP 2 161 041 A2**
(43) Date of publication of application: **10.03.2010**
(21) Application number: 09251953.7
(22) Date of filing: 06.08.2009
(51) Int. Cl.: A61L 17/04

(54) **Yarns containing thermoplastic elastomer copolymer and polyolefin filaments**

(30) Priority: 12.08.2008 US 189845
(71) Applicant: Tyco Healthcare Group LP, North Haven, CT 06473 (US)
(72) Inventor: Hotter, Joseph, Middletown, CT 06457 (US); Cuevas, Brian, Cumming, GA 30040 (US)
(74) Representative: Soames, Candida Jane

(57) **Abstract**

Braids and surgical devices are made from yarns that include a core sheath construction with at least one yarn made from a thermoplastic elastomer and at least one yarn made from a polyolefin material.

## Description

### CROSS-REFERENCE TO RELATED APPLICATION

The present application claims the benefit of and priority to U.S. Patent Application Publication No. 20070260279, the entire disclosure of which is incorporated by reference herein.

### BACKGROUND

### Technical Field

The present disclosure relates to a surgical device, i.e., sutures, including at least one yam made from a thermoplastic elastomer copolymer and at least one yam made from a polyolefin material. Also disclosed are braided multifilaments suitably adapted for use as surgical devices and made from such yarns.

### Bakground of Related Art

Braided multifilaments often offer a combination of enhanced pliability, knot security and tensile strength when compared to their monofilament counterparts. The enhanced pliability of a braided multifilament is a direct consequence of the lower resistance to bending of a bundle of very fine filaments relative to one large diameter monofilament. However, a tradeoff between braid strength and pliability exists in the design of conventional braided multifilaments.

Braided multifilaments intended for the repair of body tissues should meet certain requirements: they should be substantially non-toxic, capable of being readily sterilized, they should have good tensile strength and pliability, they should also have acceptable knot-tying and knot-holding characteristics and if the braided multifilaments are of the bio-degradable variety, the degradation of the braided multifilaments should be predictable and closely controlled. When used in combination with a fixation device (e.g., bone screw or the like), the suture must be able to withstand the heat generated when the suture contacts the fixation device.

It would be advantageous to provide a braided multifilament suture that exhibits a combination of desirable characteristics.

### SUMMARY

The present disclosure describes a surgical device having a core comprising at least one filament; and a sheath disposed around the core, the sheath including a first yam comprising a thermoplastic elastomer in combination with a second yarn comprising a polyolefin. The thermoplastic elastomer is preferably a polyester-ether block copolymer. The polyester block of the polyester-ether block copolymer may include polyalkylene terephthalate, polyethylene terephthalate, polytrimethylene terephthalate or polybutylene terephthalate. The polyether block of the polyester-ether block copolymer may be a long chain poly ether glycol, polyalkylene ether glycols, poly(ethylene ether)glycol, poly(1,2- and 1,3-propylene ether)glycol, poly(tetramethylene ether)glycol, poly(1,2-butylene ether)glycol, poly(pentamethylene ether)glycol, poly(hexamethylene ether)glycol, poly(heptamethylene ether)glycol, poly(octamethylene ether)glycol, or poly(nonamethylene ether)glycol. The thermoplastic elastomer ma y be a poly(ester-ether) block which includes a polybutylene terephthalate and poly(tetramethylene ether) glycol. Additionally, the polyolefin may be an ultra high molecular weight polyethylene.

In another embodiment, the surgical device includes a core comprising at least one filament, and, a sheath disposed around the core, the sheath including a first yarn comprising a polyester-ether copolymer in combination with a second yam comprising an ultra high molecular weight polyethylene.

In some embodiments, the first yarns and the second yarns of the sheath are in a braided construction. Additionally, the first yarns and second yarns may each be multifilament yarns.

In one embodiment, the core may be about 100 weight % polyolefin. Alternatively, the core may comprise about 100 weight % polybutester. The sheath may be from about 35 weight % to about 98 weight %, and in certain embodiments from about 40 weight % to about 94 weight % polyolefin. Additionally, the sheath may be from about 2 weight % to about 65 weight %, and in certain embodiments, from about 6 weight % to about 60 weight % thermoplastic elastomer.

In some embodiments, the surgical device includes a suture in combination with a suture anchor.

### BRIEF DESCRIPTION OF THE DRAWINGS

The above and other objects, features, and advantages will become more readily apparent from the following description, reference being made to the accompanying drawings in which:
FIG. 1 is a schematic view of a heterogeneous yam in accordance with one embodiment of this disclosure;
FIGS. 2A, 2B and 2C show illustrative embodiments of braids in accordance with one embodiment of this disclosure;
FIG. 3 shows a needle-suture combination that includes a suture made with a heterogeneous braid in accordance with one embodiment of this disclosure;
FIG. 4 is a perspective view of a suture, suture anchor and associated suture anchor driver as in one embodiment described herein;
FIG. 5 is an enlarged area of detail of Fig. 4;
FIG. 6 is a perspective view of a two part suture anchor being assembled with sutures of the present disclosure;
FIG. 7 is a perspective view of the suture anchor of FIG. 6 being positioned on an anchor driver;
FIG. 8 is a perspective view, partially shown in section, of the suture driver being rotated to drive the suture anchor carrying sutures in accordance with the present disclosure into bone; and
FIG. 9 is a cross-sectional view partially shown in perspective of the suture anchor and associated sutures installed through tissue and into bone; and,
FIG. 10 is a cross-sectional view of a core sheath construction of one embodiment of a suture according to the present disclosure.

### DETAILED DESCRIPTION

Filaments made from a thermoplastic elastomer copolymer and filaments made from a polyolefin material are used in accordance with the present disclosure to prepare yarns that can be incorporated into a braided, knitted, woven or other structure to provide a surgical device.

A plurality of filaments is used to form a yarn. A plurality of yarns is used to form a braid, knit or weave.

A "heterogeneous yarn" is a configuration containing at least two dissimilar filaments mechanically bundled together to form a yam. The filaments are continuous and discrete, so therefore each filament extends substantially along the entire length of the yarn and maintains its individual integrity during yarn preparation, processing and use.

Unlike a heterogeneous yam, a "homogeneous" yam is a configuration containing substantially similar filaments. The filaments are also continuous and discrete. Therefore each filament extends substantially along the entire length of the yarn and maintains its individual integrity during yam preparation, processing and use.

A " heterogeneous b raid" is a configuration containing at least two dissimilar yarns. The two types of yarns are intertwined in a braided construction. The yarns are continuous and discrete, so therefore each yam extends substantially along the entire length of the braid and maintains its individual integrity during braid preparation, processing and use. A heterogeneous braid may comprise either homogeneous or heterogeneous yarns.

In the broadest sense, this disclosure contemplates yarns that include at least one yam made from a thermoplastic elastomer copolymer and at least one yam made from a polyolefin material. This disclosure further contemplates the medical devices in a core sheath construction made from these yarns and their use in surgery.

It is envisioned that any thermoplastic elastomer copolymer known to one skilled in the art and capable of being spun into continuous filaments may be used. Particularly useful thermoplastic elastomer copolymers include polyester-ether block copolymers that contain at least a polyester hard segment and a polyether soft segment. These polyester-ether block copolymers can be formed of various configurations, i.e., AB, ABA, or BAB, wherein A is a polyester and B is a polyether.

Some examples of suitable polyesters include, but are not limited, to polyalkylene terephthalate, polyethylene terephthalate, polytrimethylene terephthalate and polybutylene terephthalate. Some examples of suitable polyethers include, but are not limited to, long chain poly ether glycols, polyalkylene ether glycols, poly(ethylene ether)glycol, poly(1,2- and 1,3-propylene ether)glycol, poly(tetramethylene ether)glycol, poly(1,2-butylene ether)glycol, poly(pentamethylene ether)glycol, poly(hexamethylene ether)glycol, poly(heptamethylene ether)glycol, poly(octamethylene ether)glycol, and poly(nonamethylene ether)glycol. A particularly useful polyester-ether block copolymer contains polybutylene terephthalate and a long chain polyether glycol and is commercially available under the trademark ARNITEL® from DSM, The Netherlands. In certain embodiments, the thermoplastic elastomer is comprised of a long chain ether glycol and polybutylene terephthalate, which, as used herein, is termed a "polybutester."

It is also envisioned that any polyolefin known to one skilled in the art and capable of being spun into continuous filaments may be used. In useful embodiments the polyolefin material is made from polyethylene. In particularly useful embodiments, the polyethylene material is a gel-spun ultra high molecular weight polyethylene. Ultra high molecular weight polyethylene ("UHMWP") is a linear polymer with an average molecular weight greater than about 400,000, typically in the range from about 500,000 to about 6,000,000. Gel-spun UHMWP has a high tenacity and low elongation rate to provide articles with greatly increased strength and decreased elongation.

Ultra high molecular weight polyethylene, which may be gel-spun, typically exhibits a very substantial degree of crystalline orientation (95-99%) and crystalline content (60-85%). The significant strength and stability of UHMWP is normally caused by the high degree of molecular orientation. As a result, the filaments exhibit strengths from about 375 kpsi (thousands of pounds per square inch) to about 560 kpsi, and tensile moduli of about 15 msi (millions of pounds per square inch) to about 30 msi. Gel-spun UHMWP is commercially available under the trademark SPECTRA® from Allied-Signal Technologies, Petersberg, Va. and under the trademark DYNEEMA® from DSM High Performance Fibers, JH Heerlen, The Netherlands.

The yarns may optionally contain filaments made of other materials. Materials used to construct these optional filaments can include a wide variety of natural and synthetic materials such as any materials previously known for the construction of sutures, meshes, sternal closure devices, cables, tapes and tethers. Such materials include bioabsorbable, non-bioabsorbable, biodegradable, non-biodegradable and bioerodible polymeric materials. Additionally carbon fibers, steel fibers, memory-shape alloys, silk, cotton, linen and other fibrous materials can also be employed.

Representative natural biodegradable polymers include polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers.

Representative synthetic polymer blocks include polyphosphazenes, poly (vinyl alcohols), polyamides, polyester amides, poly(amino acid)s, synthetic poly(amino acids), polyanhydrides, polycarbonates, polyacrylates, polyalkylenes, polyacrylamides, polyalkylene glycols, polyalkylene oxides, polyvinyl halides, polyvinylpyrrolidone, polylactides, polyglycolides, polysiloxanes, polyurethanes and copolymers thereof.

Examples of suitable polyacrylates include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate) and poly(octadecyl acrylate).

Synthetically modified natural polymers include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate and cellulose sulfate sodium salt. These are collectively referred to herein as "celluloses".

Representative synthetic degradable polymers include polyhydroxy acids, such as polylactides, polyglycolides and copolymers thereof; poly(hydroxybutyric acid); poly(hydroxyvaleric acid); poly(lactide-co-(ε-caprolactone-)); poly(glycolide-co-(ε-caprolactone)); polycarbonates, poly(pseudo amino acids); poly(amino acids); poly(hydroxyalkanoate)s; polyanhydrides; polyortho esters; and blends and copolymers thereof.

Examples of non-biodegradable polymers include ethylene vinyl acetate, poly(meth)acrylic acid, polyamides, polystyrene, polyvinyl chloride, polyvinylphenol, and copolymers and mixtures thereof.

Rapidly bioerodible polymers such as poly(lactide-co-glycolide)s, polyanhydrides, and polyorthoesters, which have carboxylic groups exposed on the external surface as the smooth surface of the polymer erodes, also can be used.

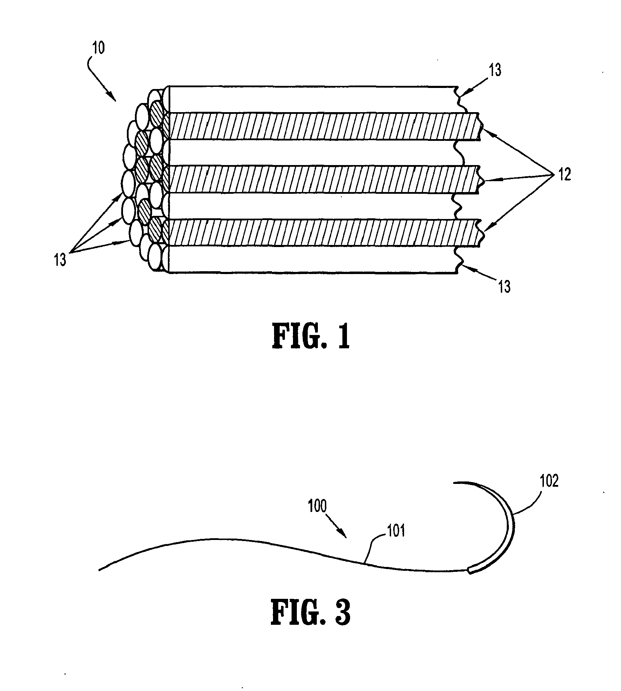

In one embodiment, a heterogeneous yam 10 contains a plurality of two dissimilar filaments as shown in Figure 1. First filaments 12 are made from a thermoplastic elastomer copolymer and second filaments 13 are made from a polyolefin material. A plurality of the two dissimilar filaments are commingled to form a heterogeneous yarn.

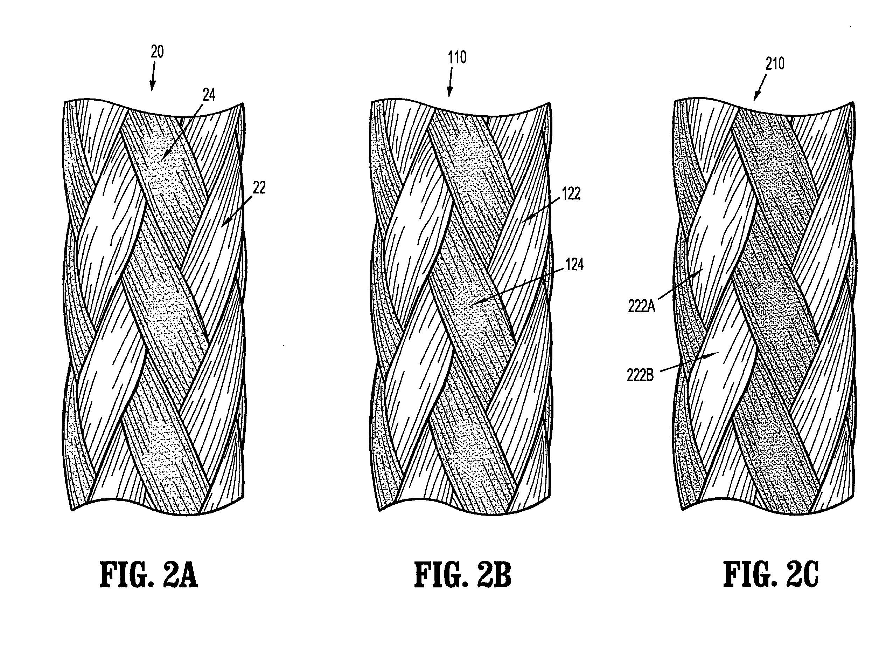

In another embodiment shown in Figure 2A, a heterogeneous braid 20 contains two dissimilar yarns. A first yam 22 contains a plurality of filaments made from a thermoplastic elastomer copolymer. A second yarn 24 contains a plurality of filaments made from a polyolefin material. The first and second yarns are intertwined to form a heterogeneous braid.

In still another embodiment shown in Figure 2B, a heterogeneous braid 120 contains a heterogeneous yarn 122 and a homogeneous yarn 124. As described above, a heterogeneous yarn contains a plurality of two dissimilar filaments. Preferably, a first filament is made from a thermoplastic elastomer copolymer and a second filament is made from a polyolefin material. A homogeneous yarn contains a plurality of filaments made from any material capable of being spun into a filament. The heterogeneous yarn and the homogeneous yarn are intertwined to form a heterogeneous braid.

In yet another embodiment shown in Figure 2C, a braid 210 contains two similar heterogeneous yarns 222A, 222B. Each heterogeneous yarn contains a plurality of two dissimilar filaments. Preferably, a first filament is made from a thermoplastic elastomer copolymer and a second filament is made from a polyolefin material. The heterogeneous yarns are intertwined to form a braid.

A heterogeneous braid and/or yarn can be prepared using conventional braiding technology and equipment commonly used in the textile industry, and in the medical industry for preparing multifilament sutures. Suitable braid constructions are disclosed, for example, in U.S. Pat. Nos. 3,187,752; 3,565,077; 4,014,973; 4,043,344; 4,047,533; 5,019,093; and 5,059,213, the disclosures of which are incorporated herein by reference. Illustrative flat braided structures (suitable, e.g., for tendon repair) which can be formed using the presently described heterogeneous yarns include those described in U.S. Patent Nos. 4,792,336 and 5,318,575. Suitable mesh structures are shown and described, for example, in Hain et al. U.S. Patent No. 5,292,328. In addition, shape memory fibers may be incorporated into non-woven structures, such as felt. One suitable non-woven structure is shown and described in Koyfman et al. U.S. Patent No. 5,393,534.

In some embodiments, core sheath constructions are preferred. For example, several of the above-mentioned U.S. Patents for braided constructions discuss core sheath structures. For example, U.S. Patent Nos. 5,019,093 discloses braided sutures and a core component around which the braid is constructed.

In some embodiments, core sheath braids include a core which may be a multifilament yam, a plurality of multifilament yarns, or a single filament. Yarns and filaments comprising the core may be either heterogeneous or homogeneous. In certain embodiments, the core may comprise a first yam and a second yarn which are dissimilar, although in other embodiments, the first and second yarns may be similar. The first yarns and the second yarns may be multifilament yarns which may be homogeneous or heterogeneous. Multifilament yarns of the present disclosure may be constructed by braiding, plying, weaving, or twisting individual filaments together. Alternately, the core may comprise at least two multifilament yarns, and the multifilament yarns may be woven, braided or otherwise entangled together.

In some embodiments, core sheath braids include a sheath which may be a multifilament yarn or a plurality of multifilament yarns. Similar to the core described above, yarns comprising the sheath may be either heterogeneous or homogeneous. In certain embodiments, the sheath comprises first yarns and second yarns which are dissimilar, although in other embodiments, the first and second yarns may be of the same material. Additionally, the first yarn may comprise UHMWP and the second yam may be a colored identifier, i.e. a tracer, such as silk or nylon. The first yarns and the second yarns may be multifilament yarns which may be homogeneous or heterogeneous. Multifilament yarns of the present disclosure may be constructed by braiding, plying, weaving, or twisting individual yarns together. Alternately, the sheath may comprise a plurality of multifilament yams; the plurality of multifilament yarns may be woven, braided or otherwise entangled together. For example, a suture may include a core, which is a twisted homogeneous yarn, and a braided multifilament sheath, having a first thermoplastic elastomer yam and second polyolefin yam disposed about the core.

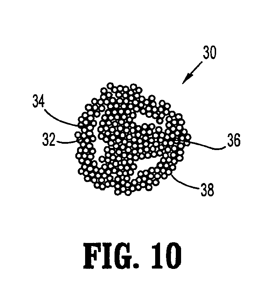

In another example, the core sheath construction may include a multifilament core yarn in combination with a multifilament sheath as shown in Figure 10. The suture 30 includes a core 36 which is a multifilament yarn although is it contemplated that the core may a monofilament. The suture 30 includes a multifilament sheath 38 which has a plurality of two dissimilar yarns 32 and 34, disposed about the core 36.

The core may comprise about 100 % of a polyolefin material. The core is from about 0 weight % to about 30 weight % of the suture, and in certain embodiments, from about 5 weight % to about 25 weight % of the suture. The sheath yarns comprise from about 70% to about 100% by weight of the suture, and in certain embodiments, from about 75% to 95% by weight of the suture.

As described above, the sheath may contain two dissimilar yarns, the first yam being a thermoplastic elastomer copolymer and the second yam being a polyolefin. More specifically, in some embodiments, the sheath is about 35 weight % to about 98 weight % polyolefin, and in certain embodiments, from about 40 weight % to about 94 weight % polyolefin. Additionally, the sheath is about 2 weight % to about 65 weight % thermoplastic elastomer, and in certain embodiments from about 6 weight % to about 60 weight % thermoplastic elastomer.

Overall, the composition of the suture may contain from about 2 weight % to about 75 weight %, and in some embodiments from about 5 weight % to about 65 weight percent of a thermoplastic elastomer. The polyolefin composition in the suture is from about 25 weight % to about 98 weight %, and in certain embodiments, from about 35 weight % to about 95 weight %.

Particularly useful sheath materials may include a thermoplastic elastomer such as ARNITEL®, and a polyolefin, such as an ultra high molecular weight polyethylene (UHMWP) like DYNEEMA®. A particularly useful core material may be a polyolefin, such as an ultra high molecular weight polyethylene like DYNEEMA ®. Alternatively, the core material may comprise a thermoplastic elastomer such as ARNITEL®.

Furthermore, sutures of the present disclosure may include a colored identifier such as a tracer. "Tracers" as defined herein, include an identifiable trace such as a filament which provides color contrasting to the suture or medical device against a surrounding environment such as the surgical field. For example, the tracer may be a dyed, pigmented, or color coated polymer filament of a material such as polybutester or nylon.

The tracers may be comingled or braided with other yarns to create a multifilament yam. A length or portion of the suture may include a tracer, or conversely, a tracer may run the entire length of the suture. The tracers may be braided into the suture to exhibit a specific pattern on the surface of the suture, which may assist a surgeon in identifying specific suture strands in the surgical field.

If desired, the surface of a filament, yam or braid can be coated with a bioabsorbable or nonabsorbable coating to further improve the performance of the braid. For example, a braid can be immersed in a solution of a desired coating polymer in an organic solvent, and then dried to remove the solvent.

If the surface of a filament, yam or braid is coated, then the coating composition may desirably contain bioactive materials. Some examples include: vasoactive agents, neuroactive agents, hormones, growth factors, cytokines, anaesthetics, steroids, anticoagulants, anti-inflammatories, immunomodulating agents, cytotoxic agents, prophylactic agents, antibiotics, antimicrobials, antivirals, antisense, antigens and antibodies.

A heterogeneous braid is sterilized so it can be used for a host of medical applications, especially for use as a surgical suture, cable, tether, tape and sternal closure device, preferably attached to a needle, suture anchor, or bone anchor. For example, as shown in Figure 3, a needle-suture combination 100 includes a suture 101 made from a heterogeneous yarn in accordance with this disclosure attached to a needle 102. A braid can be sterilized using any of the conventional techniques well known in the art.

Once sterilized, a braided multifilament surgical device, as described herein, may be used to repair wounds located between two or more soft tissues, two or more hard tissues, or at least one soft tissue and at least one hard tissue. The braided multifilament surgical device is passed through, wrapped around or secured to tissue and then the tissue is approximated by manipulating the braided multifilament surgical device, such as, for example, by tying a knot, cinching the device, applying a buckle, or the like.

Sutures made in accordance with the foregoing description will exhibit superior strength and resistance to abrasion, and may find particular use in cardiac surgery and orthopedic surgery. With respect to orthopedic surgery in particular, the suture will be useful in securing bone under high stress and abrasion. The present multifilament braided sutures can advantageously be used in combination with a fixation device, including, but not limited to, for example, suture anchors.

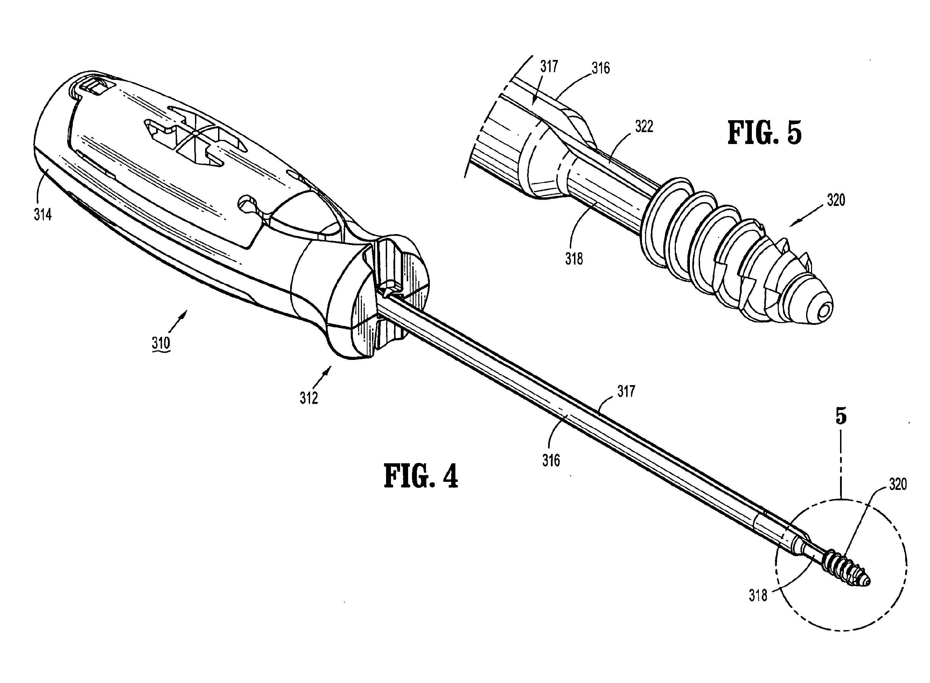

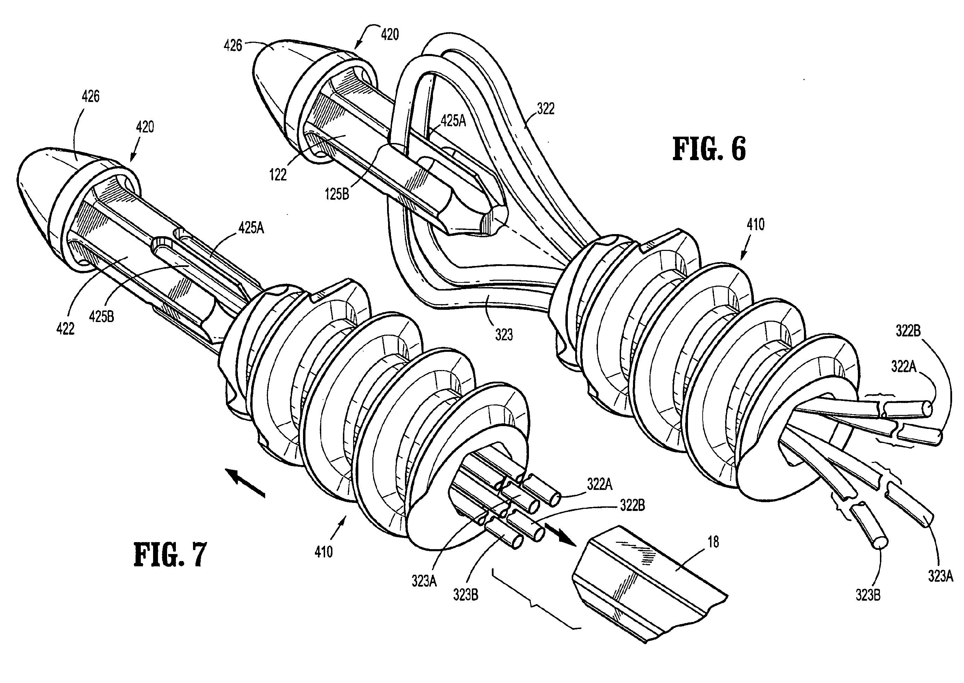

In a particularly useful embodiment, it is contemplated that the suture in accordance with the disclosure may be delivered in conjunction with a suture anchor delivery system and may be passed through tissue using an arthroscopic suturing instrument. Referring now to Figs. 4 and 5, one suitable suture anchor delivery system 310 is shown having a handle 314 with an elongate shaft 316 supporting a threaded suture anchor 320 at the distal tip 318 of the shaft 316 away from the handle 314. As shown in Fig. 5, suture 322 made in accordance with the present disclosure is attached to the suture anchor 320 and is led through trough 317 in the shaft 316 (and a corresponding trough (not shown) on the other side shaft 316) to handle 314. Referring now to Figs. 6 and 7, one method of pre-attaching a pair of sutures 322, 323 to a suture anchor is shown. As shown, suture anchor 320 consists of two parts, a hollow-threaded body portion 410 and a tip portion 420 having a shaft 422 insertable into the hollow body portion 410 and configured to receive and hold two sutures 322, 323 through transverse apertures 425A, 425B, as shown. Enlarged tip bead section 426 does not pass into or through the hollow threaded body 410, thereby retaining the suture relative to the suture anchor as the sutures 322, 323 are placed under tension by pulling on proximal ends 322A, 322B, 323A, 323B. Of course, numerous other types of suture anchors and methods of attaching sutures and in accordance with the present disclosure will occur to those skilled in the art. By way of example, the suture alternatively may be attached to a push-in-type anchor rather than a screw-in type anchor. See for example, Larsen U.S. Patent No. 5,993,459. Preferably, the proximal ends of the suture are attached to needles (not shown) suitable for use during surgery to pass the suture through tissue and, via appropriate manipulation (e.g., knot tying and/or cinching) secure the tissue relative to the anchor.

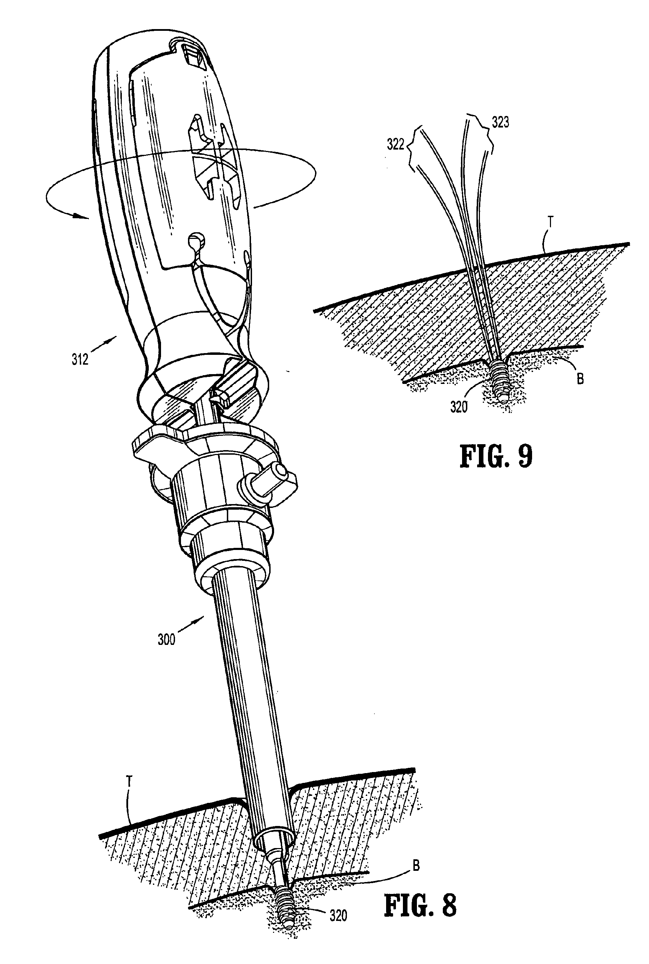

In use during an arthroscopic procedure a cannula 300 is inserted into the joint capsule and the shaft 316 of the suture anchor delivery system is inserted through the cannula 300 to a prepared site suitable to receive suture anchor. Fig. 8 shows the shaft of the instrument inserted through cannula 430 with the suture anchor 320 inserted into bone B. The suture anchor 320 is released from the delivery system 310 leaving the sutures 322, 323 available for manipulation to secure the soft tissue T to bone B. In a further embodiment the sutures are attached to needles (not shown) suitable for passing through soft tissue. The needles may be traditional suture needles suitable for use with an arthroscopic suturing instrument. One such instrument is the Arthrosew instrument (U.S.S. Sports Medicine, North Haven, CT) which utilizes a double ended surgical incision mender. Most preferably, the handle portion of the suture anchor delivery system includes releasable suture management members (not shown) which hold the suture needles for use. If the suture needles are to be used with a suturing device, the suture management members are configured to engage the suturing instrument in a suitable manner to transfer control of the needle to the suturing instrument. The needles and suture(s) are passed through soft tissue and the suture is manipulated, such as by forming in the suture, to secure the soft tissue relative to the suture anchor. Thereafter, the patient is closed in a suitable manner depending upon whether the procedure was conducted as an open, mini-open or closed arthroscopic approach.

In the context of a suture anchor, a suture constructed in accordance with the present disclosure provides significantly enhanced resistance to abrasion as the suture is manipulated, including drawing the suture through the suture eyelets of the suture anchor, forming knots in the suture, and cinching the knots down tightly for secure approximation of the soft tissue to bone.

### EXAMPLE 1

### USP Size 2

The composite suture has a sheath of ultra high molecular weight polyethylene (UHMWP) multifilament yam and polybutester multifilament yam. The polybutester multifilament yam is blue in color, the polybutester includes the addition of Heliogen® Blue K7090 pigment, commercially available from BASF. The suture is made on a conventional Ratera braider. The sheath contains about 76 weight % UHMWP and about 24 weight % polybutester. The core of the suture consists of about 100 weight % UHMWP yarns. Loaded onto the braider are 8 carriers of 110 dtex ultra high molecular weight polyethylene multifilament yarns in the clockwise direction, 4 carriers of 110 dtex UHMWP multifilament yarns in the counter clockwise direction and 4 carriers of 106 dtex polybutester multifilament yarns in the counterclockwise. The counterclockwise bobbins are loaded in a 2x2x2x2 manner with two consecutive carriers of UHMWP alternated with two consecutive carriers of polybutester yarns. The braid has a core construction of 1 ply 220 dtex UHMWP. The braider pick count is set at between 51 - 55 ppi, the core tension is set between 70 - 90 grams and the take-up tension at approximately 300 grams.

Of the overall suture, the polybutesters in the sheath makes up approximately 22 weight % of the suture and the UHMWP in the sheath makes up approximately 68 weight % of the suture. The UHMWP in the core makes up approximately 10 weight % of the suture.

### EXAMPLE 2

### USP Size 2

The composite suture has a sheath of ultra high molecular weight polyethylene (UHMWP) multifilament yam and polybutester monofilament yam. The suture was made on a conventional Ratera braider. The sheath contained about 94 weight % UHMWP and about 6 weight % polybutester, the polybutester was colored blue by the inclusion of Heliogen® Blue K 7090 pigment, commercially available from BASF. The core of the suture consisted of 100 weight % UHMWP multifilament yam. Loaded onto the braider were 8 carriers of 110 dtex UHMWP multifilament yarns in the clockwise direction, 6 carriers of 110 dtex UHMWP multifilament yarns in the counter clockwise direction and 2 carriers of 50 dtex polybutester monofilament yarns in the counterclockwise direction. The counterclockwise bobbins were loaded in a 3x1x3x1 manner with three consecutive carriers of UHMWP alternated with one carrier of polybutester yarn. The braid had a core construction of 1 ply 220 dtex UHMWP. The braider pick count was set at between 51 - 55 ppi, the core tension was set between 70 - 90 grams and the take-up tension at approximately 300 grams.

Of the overall suture, the polybutesters in the sheath makes up approximately 6 weight % of the suture and the UHMWP in the sheath makes up approximately 84 weight % of the suture. The UHMWP in the core makes up approximately 10 weight % of the suture.

Various modifications and variations of the yarns, braids and devices and uses thereof will be apparent to those skilled in the art from the foregoing detailed description. Such modifications and variations are intended to come within the scope of the following claims.

## Claims

1. A surgical device comprising:
a core comprising at least one filament; and
a sheath disposed around the core, the sheath including a first yam comprising a thermoplastic elastomer and a second yarn comprising a polyolefin material.

2. The surgical device according to claim 1, wherein the thermoplastic elastomer is a polyester-ether block copolymer.

3. The surgical device according to claim 2, wherein the polyester-ether block copolymer comprises a polyester block, wherein the polyester block is selected from the group consisting of polyalkylene terephthalate, polyethylene terephthalate, polytrimethylene terephthalate and polybutylene terephthalate.

4. The surgical device according to claim 2, wherein the polyester-ether block copolymer comprises a polyether block, wherein the polyether block is selected from the group consisting of long chain poly ether glycols, polyalkylene ether glycols, poly(ethylene ether)glycol, poly(1,2- and 1,3-propylene ether)glycol, poly(tetramethylene ether)glycol, poly(1,2-butylene ether)glycol, poly(pentamethylene ether)glycol, poly(hexamethylene ether)glycol, poly(heptamethylene ether)glycol, poly(octamethylene ether)glycol, and poly(nonamethylene ether)glycol.

5. The surgical device according to claim 1, wherein the thermoplastic elastomer comprises a polyester-ether block copolymer including polybutylene terephthalate and poly(tetramethylene ether) glycol.

6. The surgical device according to claim 1, wherein the polyolefin material comprises an ultra high molecular weight polyethylene; or the surgical device according to claim 1, wherein the first yam and the second yarn are in a braided construction; or the surgical device according to claim 1, wherein the first yam and the second yam are multifilament yarns.

7. The surgical device according to claim 1, wherein the core comprises about 100 weight % polyolefin; or the surgical device according to claim 1, wherein the core comprises about 100 weight % polybutester; or the surgical device according to claim 1, wherein the sheath comprises from about 35 weight % to about 98 weight % polyolefin; or the surgical device according to claim 1, wherein the sheath comprises from about 40 weight % to about 94 weight % polyolefin; or the surgical device according to claim 1, wherein the sheath comprises from about 2 weight % to about 65 weight % thermoplastic elastomer; or the surgical device according to claim 1, wherein the sheath comprises from about 6 weight % to about 60 weight % thermoplastic elastomer.

8. The surgical device according to claim 1, wherein the surgical device comprises a suture in combination with a suture anchor; or the surgical device according to claim 1, further including a colored identifier.

9. A surgical device comprising:
a core comprising at least one filament; and,
a sheath disposed around the core, the sheath including a first yam comprising a polyester-ether copolymer in combination with a second yam comprising an ultra high molecular weight polyethylene.

10. The surgical device according to claim 9, wherein the first yarn and the second yarn are multifilament yarns; or the surgical device according to claim 9, wherein the polyester-ether copolymer comprises a polybutester.

11. The surgical device according to claim 9, wherein the core comprises about 100 weight % ultra high molecular weight polyethylene; or the surgical device according to claim 9, wherein the core comprises about 100 weight % polybutester; or the surgical device according to claim 9, wherein the sheath comprises from about 35 weight % to about 98 weight % ultra high molecular weight polyethylene; or the surgical device according to claim 9, wherein the sheath comprises from about 40 weight % to about 94 weight % ultra high molecular weight polyethylene; or the surgical device according to claim 9, wherein the sheath comprises from about 2 weight % to about 65 weight % polyester-ether copolymer; or the surgical device according to claim 9, wherein the sheath comprises from about 6 weight % to about 60 weight % polyester-ether copolymer.

12. The surgical device according to claim 9, further including a colored identifier.
